# EUROPÄISCHE PATENTANMELDUNG

(11) **EP 3 326 592 A1**
(43) Veröffentlichungstag der Anmeldung: **30.05.2018**
(21) Anmeldenummer: 16200333.9
(22) Anmeldetag: 23.11.2016
(51) Int. Cl.: A61F 9/06

(54) **SCHUTZKASSETTE**

(71) Anmelder: optrel Holding AG, 9050 Appenzeil (CH)
(72) Erfinder: HOFER KRANER, Ramon, 9100 Herisau (CH)
(74) Vertreter: Daub, Thomas

(57) **Zusammenfassung**

Schutzkassette für eine Blendschutzvorrichtung (12a; 12b), mit einem optischen Blendschutzfilter (14a; 14b), welcher zumindest eine Flüssigkristallzelle (16a; 16b) aufweist, mit zumindest einer Steuer- und/oder Regeleinheit (18a; 18b), welche dazu vorgesehen ist, abhängig von einem erfassten Arbeitszustand und/oder einer Lichteinstrahlung eine Durchlässigkeit des optischen Blendschutzfilters (14a; 14b) zu steuern und/oder zu regeln, und mit zumindest einer Anzeigeeinheit (20a; 20b) zu einer Anzeige von Betriebs- und/oder Prozessparametern eines externen Schweißgeräts (22a; 22b) und/oder eines Schweißverfahrens.

Es wird vorgeschlagen, dass die zumindest eine Anzeigeeinheit (20a; 20b) zumindest eine abstrahierte Symbolanzeige (24a, 26a, 52a, 54a; 24b) aufweist, welche zumindest während eines Betriebs in einem peripheren Sichtfeld eines Bedieners angeordnet ist.

## Beschreibung

### Stand der Technik

Die Erfindung betrifft eine Schutzkassette.

Es ist bereits eine Schutzkassette für eine Blendschutzvorrichtung, mit einem optischen Blendschutzfilter, welcher zumindest eine Flüssigkristallzelle aufweist, mit zumindest einer Steuer- und/oder Regeleinheit, welche dazu vorgesehen ist, abhängig von einem erfassten Arbeitszustand und/oder einer Lichteinstrahlung eine Durchlässigkeit des optischen Blendschutzfilters zu steuern und/oder zu regeln, und mit zumindest einer Anzeigeeinheit zu einer Anzeige von Betriebs- und/oder Prozessparametern eines externen Schweißgeräts und/oder eines Schweißverfahrens, vorgeschlagen worden. Entsprechende Anzeigeeinheiten sind in der Regel Digitalanzeigen, bei welchen die Betriebs- und/oder Prozessparameter in Zahlen wiedergegeben werden. Die Anzeigeeinheiten müssen in ausreichendem Abstand zum Auge angebracht werden oder es muss eine Optik vorgesehen werden, um eine gute Lesbarkeit zu erreichen. Eine beabstandete Anordnung der Anzeigeeinheit hat auch eine beabstandete Anordnung der Schutzkassette zur Folge, was zu einer Einschränkung des Sichtfelds führt.

Die Aufgabe der Erfindung besteht insbesondere darin, eine gattungsgemäße Vorrichtung mit verbesserten Eigenschaften hinsichtlich einer Ablesbarkeit bereitzustellen, insbesondere ohne ein Sichtfeld einzuschränken. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einer Schutzkassette für eine Blendschutzvorrichtung, mit einem optischen Blendschutzfilter, welcher zumindest eine Flüssigkristallzelle aufweist, mit zumindest einer Steuer- und/oder Regeleinheit, welche dazu vorgesehen ist, abhängig von einem erfassten Arbeitszustand und/oder einer Lichteinstrahlung eine Durchlässigkeit des optischen Blendschutzfilters zu steuern und/oder zu regeln, und mit zumindest einer Anzeigeeinheit zu einer Anzeige von Betriebs- und/oder Prozessparametern eines externen Schweißgeräts und/oder eines Schweißverfahrens.

Es wird vorgeschlagen, dass die zumindest eine Anzeigeeinheit zumindest eine abstrahierte Symbolanzeige aufweist, welche zumindest während eines Betriebs in einem peripheren Sichtfeld eines Bedieners angeordnet ist. Vorzugsweise ist der Bediener insbesondere von einem Träger der Blendschutzvorrichtung gebildet. Bevorzugt ist die zumindest eine abstrahierte Symbolanzeige in einem getragenen Zustand der Blendschutzvorrichtung in einem unfokusierbaren Sichtfeld des Bedieners angeordnet. Vorzugsweise ist die Schutzkassette in einem getragenen Zustand gegenüber den Augen des Bedieners positionsfest. Unter einer "Schutzkassette" soll in diesem Zusammenhang insbesondere ein vorzugsweise zusammenhängendes Modul der Blendschutzvorrichtung verstanden werden. Vorzugsweise weist die Schutzkassette insbesondere zumindest teilweise ein gemeinsames Gehäuse auf, in und/oder an welchem zumindest ein wesentlicher Teil der Bauteile der Schutzkassette angeordnet sind. Bevorzugt soll darunter insbesondere ein eigenständiges Modul verstanden werden, welches vorzugsweise selbstständig funktionsfähig ist. Grundsätzlich wäre jedoch auch denkbar, dass die Schutzkassette in einer weiteren Einheit der Blendschutzvorrichtung, wie beispielsweise einer Schildeinheit, integriert ist. Ferner soll in diesem Zusammenhang unter einer "Blendschutzvorrichtung" insbesondere eine Vorrichtung verstanden werden, welche zu einem Schutz eines Benutzers vor zu großer Helligkeit und/oder Funken vorgesehen ist. Vorzugsweise soll darunter insbesondere eine Vorrichtung verstanden werden, welche zu einem Schutz von Augen und/oder einem Gesichtsbereich eines Benutzers während eines Schweiß- und/oder Schleifprozesses dient. Bevorzugt soll darunter insbesondere eine Vorrichtung verstanden werden, welche insbesondere zu einem Schutz der Augen eines Benutzers zumindest während eines Schweißprozesses dient. Es sind verschiedene, einem Fachmann als sinnvoll erscheinende Ausgestaltungen einer Schutzvorrichtung denkbar, wie beispielsweise als Schweißhelm, -schirm, -maske und/oder -schild. Ferner sind verschiedene, einem Fachmann als sinnvoll erscheinende Flüssigkristallzellen denkbar, wie insbesondere eine TN-Flüssigkristallzelle mit der Twisted-Nematic-Technik. Grundsätzlich wären jedoch auch andere, einem Fachmann als sinnvoll erscheinende Ausbildungen der Flüssigkristallzellen denkbar, wie beispielsweise als STN-Flüssigkristallzellen mit der Super-Twisted-Nematic-Technik, DSTN-Flüssigkristallzellen mit der Double-Super-Twisted-Nematic-Technik, TSTN-Flüssigkristallzellen mit der Triple-Super-Twisted-Nematic-Technik, VA-Flüssigkristallzellen mit der Vertical-Alignment-Technik, insbesondere PVA/MVA-Flüssigkristallzellen mit der Patterned-Vertical-Alignment- und/oder Multi-Domain-Vertical-Alignment-Technik, IPS-Flüssigkristallzellen mit der In-Plane-Switching-Technik, FLCD-Flüssigkristallzellen, also ferroelektrische Flüssigkristallzellen, und/oder TN-Flüssigkristallzellen mit der Guest-Host-Technik. Unter einem "optischen Blendschutzfilter" soll insbesondere ein optischer Filter für eine Blendschutzvorrichtung verstanden werden, welcher insbesondere ein Schutzglas und/oder ein Kunststoffschutzglas bildet. Vorzugsweise soll darunter insbesondere ein optischer Filter verstanden werden, dessen Lichtdurchlässigkeit einstellbar ausgeführt ist. Bevorzugt soll darunter insbesondere ein optischer Schweißschutzfilter mit einer automatischen Verdunkelung verstanden werden. Besonders bevorzugt weist der Blendschutzfilter zumindest eine in der Transmission schaltbare Flüssigkristallebene auf. Es sind verschiedene, einem Fachmann als sinnvoll erscheinende Ausgestaltungen des optischen Blendschutzfilters denkbar, insbesondere soll darunter jedoch ein ADF, auch "automatic darkening filter" oder "automatischer Schweißerschutzfilter" genannt, verstanden werden. Des Weiteren soll unter einer "Steuer- und/oder Regeleinheit" in diesem Zusammenhang insbesondere eine Einheit mit zumindest einer Steuerelektronik verstanden werden. Unter einer "Steuerelektronik" soll insbesondere eine Einheit mit zumindest einer elektronischen Schaltung verstanden werden, welche vorzugsweise aus Spannungs- und Vergleichsregelbausteinen besteht. Grundsätzlich kann die Steuerelektronik jedoch auch komplexer aufgebaut sein, wie insbesondere durch die Nutzung einer anwendungsspezifischen integrierten Schaltung (ASIC) und/oder eines Mikrokontrollerbausteins.

Unter einer "Anzeigeeinheit" soll in diesem Zusammenhang insbesondere eine Einheit verstanden werden, welche zu einer Anzeige, insbesondere zu einer Ausgabe, einer Information vorgesehen ist. Vorzugsweise ist die Einheit zu einer Ausgabe einer Information an den Bediener vorgesehen. Bevorzugt umfasst die Anzeigeeinheit zumindest ein Anzeigemittel, insbesondere zumindest ein optisches Anzeigemittel. Unter einem "Anzeigemittel" soll insbesondere ein Mittel verstanden werden, das zumindest zwei Anzeigezustände aufweist und in zumindest einem Anzeigezustand eine optische und/oder akustische Anzeige vermittelt und vorzugsweise ein für einen Menschen sichtbares und/oder hörbares Signal abgibt. Eine optische Anzeige kann dabei sowohl direkt über das optische Anzeigemittel erfolgen als auch beispielsweise von einer Reflektion gebildet sein, in welcher das optische Anzeigemittel reflektiert wird. Bei einer Reflektion ist die für einen Bediener sichtbare Anzeige insbesondere von der Reflektionsoberfläche gebildet. Unter einem "optischen Anzeigemittel" soll insbesondere ein Leuchtmittel, vorzugsweise eine LED, und/oder eine, vorzugsweise hinterleuchtete, Displayeinheit, insbesondere eine Matrixdisplayeinheit, vorzugsweise ein LCD-Display, ein OLED-Display und/oder elektronisches Papiers (e-paper, E-Ink), verstanden werden. Ferner soll in diesem Zusammenhang unter einem "Betriebs- und/oder Prozessparameter eines externen Schweißgeräts und/oder eines Schweißverfahrens" insbesondere ein, vorzugsweise variabler, Betriebs- und/oder Prozessparameter eines sich in einer Umgebung der Blendschutzvorrichtung befindlichen Schweißgeräts und/oder eines von dem Schweißgerät durchgeführten Schweißverfahren verstanden werden. Vorzugsweise soll darunter insbesondere ein variabler Betriebs- und/oder Prozessparameter eines mit der Blendschutzvorrichtung gekoppelten Schweißgeräts verstanden werden. Vorzugsweise ist die Blendschutzvorrichtung dabei insbesondere mit einem Schweißgerät gekoppelt, mit welchem der Bediener arbeitet. Es sind verschiedene, einem Fachmann als sinnvoll erscheinende Betriebs- und/oder Prozessparameter eines Schweißgeräts und/oder eines Schweißverfahrens denkbar, wie beispielsweise ein Strom, eine Spannung, ein Drahtvorschub, ein Gasfluss, eine Temperatur, ein Arbeitsfortschritt, ein Prozess, ein Zustand des Schweißgeräts, wie beispielsweise die aktuelle freie Drahtelektrodenlänge (Stickout), und/oder ein aktuell eingestelltes Programm des Schweißgeräts.

Unter einer "abstrahierten Symbolanzeige" soll in diesem Zusammenhang insbesondere eine durch ein optisches Anzeigemittel der Anzeigeeinheit erzeugte Anzeige verstanden werden, bei welchem Informationen abstrahiert und/oder für einen Bediener in abgeleiteter Form dargestellt werden. Vorzugsweise soll darunter insbesondere eine durch das Anzeigemittel erzeugte Anzeige verstanden werden, über welche Informationen zu einem Ablesen von einem Bediener verarbeitet werden müssen. Bevorzugt sind die Informationen zumindest teilweise codiert. Besonders bevorzugt soll darunter insbesondere eine Anzeige verstanden werden, bei welchem Informationen insbesondere über Formen, Farben und/oder Positionen, insbesondere der Formen und/oder Farben, angezeigt werden. Vorzugsweise findet bei der abstrahierten Symbolanzeige eine grafische Darstellung von Informationen statt. Bevorzugt werden bei der abstrahierten Symbolanzeige Informationen insbesondere zumindest im Wesentlichen frei von Zahlen, Zeichen und Buchstaben dargestellt. Unter einem "peripheren Sichtfeld des Bedieners" soll in diesem Zusammenhang insbesondere ein Randbereich des Sichtfelds des Bedieners verstanden werden. Vorzugsweise soll darunter insbesondere ein Teil des Sichtfelds des Bedieners verstanden werden, welcher außerhalb der Gesichtslinie der Augen des Bedieners liegt. Bevorzugt soll darunter insbesondere ein Teil des Sichtfelds des Bedieners verstanden werden, bei welchem eine Wahrnehmung durch neben der Fovea gelegene Areale der Netzhaut erfolgt. Vorzugsweise soll darunter insbesondere ein Teil des Sichtfelds des Bedieners verstanden werden, welcher außerhalb des zentralen Sichtfelds des Bedieners angeordnet ist und insbesondere an dieses angrenzt. Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Durch die erfindungsgemäße Ausgestaltung der Schutzkassette kann insbesondere eine Ablesbarkeit der Anzeigeeinheit erreicht werden. Insbesondere kann erreicht werden, dass die Schutzkassette vorteilhaft nah vor einem Gesicht des Bedieners angeordnet werden kann, ohne dass eine Ablesbarkeit der Anzeigeeinheit für einen Bediener eingeschränkt ist. Vorzugsweise können insbesondere Informationen in einem Blickwinkel bzw. außerhalb eines zentralen Sichtfelds des Bedieners ablesbar angebracht werden. Es kann insbesondere eine Ablesbarkeit der Informationen auch in einem getragenen Zustand der Schutzkassette bzw. der Blendschutzvorrichtung gewährleistet werden, ohne ein zentrales Sichtfeld des Bedieners und damit einen Komfort einzuschränken. Es kann einem Bediener ein maximales Sichtfeld bereitgestellt werden.

Des Weiteren wird vorgeschlagen, dass die zumindest eine Anzeigeeinheit zumindest eine abstrahierte Symbolanzeige aufweist, welche von einer Reflektionsanzeige gebildet ist. Bevorzugt ist die Reflektionsanzeige von einer für einen Bediener sichtbaren Reflektion in einer Schutzscheibe der Schutzkassette gebildet. Vorzugsweise ist die zumindest eine abstrahierte Symbolanzeige von einer Reflektionsanzeige gebildet, bei welcher die für einen Bediener sichtbare Anzeige von einem bildgebenden Teil des Anzeigemittels der Anzeigeeinheit beabstandet ausgebildet ist. Bevorzugt wird bei der abstrahierten Symbolanzeige lediglich eine Ausgabe des bildgebenden Teils des Anzeigemittels der Anzeigeeinheit in ein Sichtfeld des Bedieners reflektiert. Besonders bevorzugt kann die abstrahierte Symbolanzeige, welche von einer Reflektion gebildet ist, sowohl in einem zentralen Sichtfeld des Bedieners als auch in einem peripheren Sichtfeld des Bedieners angeordnet sein. Unter einer "Reflektionsanzeige" soll in diesem Zusammenhang insbesondere eine Anzeige verstanden werden, bei welcher Informationen in ein Sichtfeld des Bedieners projiziert werden. Vorzugsweise soll darunter insbesondere eine Anzeige verstanden werden, welche von einer Reflektionsfläche gebildet ist. Bevorzugt weist die Anzeigeeinheit hierfür zumindest eine bildgebene Einheit und eine geeignete Reflektionsfläche auf, wobei eine Anzeige über die Reflektionsfläche erfolgt. Die bildgebende Einheit erzeugt dabei insbesondere das informationsenthaltende Bild, welches über die Reflektionsfläche zum Auge geleitet wird. Grundsätzlich könnte zudem ein Optikmodul vorgesehen sein, welches das Bild auf die Reflektionsfläche lenkt. Diese Reflektionsfläche ist vorzugsweise eine spiegelnde, lichtdurchlässige Scheibe. Der Bediener sieht daher insbesondere sowohl die gespiegelten Informationen der Anzeige als auch die reale Welt hinter der Scheibe. Hierdurch kann eine vorteilhafte Anzeige bereitgestellt werden. Es kann insbesondere eine vorteilhafte Ablesbarkeit der Anzeige erreicht werden.

Ferner wird vorgeschlagen, dass die zumindest eine Anzeigeeinheit zumindest zwei abstrahierte Symbolanzeigen aufweist, welche zumindest während eines Betriebs jeweils in einem peripheren Sichtfeld des Bedieners angeordnet sind. Vorzugsweise sind die abstrahierten Symbolanzeigen jeweils zu einer Anzeige von unterschiedlichen Betriebs- und/oder Prozessparametern eines externen Schweißgeräts vorgesehen. Besonders bevorzugt sind die abstrahierten Symbolanzeigen jeweils auf verschiedenen Seiten des optischen Blendschutzfilters angeordnet. Dadurch können insbesondere mehrere Informationen an der Schutzkassette dargestellt werden. Es kann insbesondere eine Ablesbarkeit mehrerer Informationen auch in einem getragenen Zustand der Schutzkassette bzw. der Blendschutzvorrichtung gewährleistet werden.

Des Weiteren wird vorgeschlagen, dass die zumindest eine abstrahierte Symbolanzeige der Anzeigeeinheit während eines Betriebs in einem unfokussierbaren Sichtfeld des Bedieners angeordnet ist. Vorzugsweise ist die zumindest eine abstrahierte Symbolanzeige der Anzeigeeinheit während eines Betriebs, zumindest bei gerader Kopfhaltung und einem direkten Geradeausschauen in einem peripheren Sichtfeld des Bedieners angeordnet. Ferner soll in diesem Zusammenhang unter einem "unfokussierbaren Sichtfeld des Bedieners" insbesondere ein Teil eines Sichtfelds des Bedieners verstanden werden, welcher aufgrund seiner geringen Distanz zum Auge und/oder aufgrund seines Winkels gegenüber einer Mittelachse des Sichtfelds nicht oder nur schwer für einen Bediener fokussierbar ist. Vorzugsweise soll darunter insbesondere ein Teil eines Sichtfelds des Bedieners verstanden werden, welcher zumindest bei gerader Kopfhaltung und einem direkten Geradeausschauen für einen Bediener unscharf erscheint. Bevorzugt soll darunter insbesondere ein Teil des Sichtfelds verstanden werden, welcher weniger als 20 cm, vorzugsweise weniger als 15 cm und besonders bevorzugt weniger als 10 cm von zumindest einem Auge des Bedieners entfernt ist. Dadurch kann erreicht werden, dass die Schutzkassette vorteilhaft nah vor einem Gesicht des Bedieners angeordnet werden kann, ohne dass eine Ablesbarkeit der Anzeigeeinheit für einen Bediener eingeschränkt ist. Vorzugsweise kann durch die abstrahierte Symbolanzeige auf eine Optik für die Ablesung der dargestellten Information verzichtet werden. Obwohl die Distanz zu kurz ist, um auf die abstrahierte Symbolanzeige zu fokussieren, können bestimmte Informationen wahrgenommen und sogar Werte abgelesen werden. Die Anzeige erscheint dann nicht klar sondern leicht verschwommen. Durch die abstrahierte Symbolanzeige kann trotzdem eine gute Lesbarkeit erreicht werden.

Es wird ferner vorgeschlagen, dass die zumindest eine abstrahierte Symbolanzeige der Anzeigeeinheit von einer Balken- oder Punktanzeige gebildet ist. Unter einer "Balkenanzeige" soll in diesem Zusammenhang insbesondere eine Anzeige eines Anzeigemittels der Anzeigeeinheit verstanden werden, bei welchem ein Balken zu einer Anzeige von Informationen dient. Der Balken kann dabei von einem kontinuierlichen oder segmentierten Balken gebildet sein. Vorzugsweise soll darunter insbesondere eine Anzeige verstanden werden, über welche Informationen mittels der Darstellung von zumindest einem Balkensegment angezeigt werden. Eine Information kann dabei beispielsweise mittels einer Skala für die Größe eines Signals, bei der sich die Länge des Balkens mit der Signalgröße verändert, angezeigt werden. Es wäre jedoch auch denkbar, dass die Informationen mittels Codierung an dem Balken dargestellt werden. Besonders bevorzugt weist ein Balken der Balkenanzeige mehrere Balkensegmente auf, welche zu einer Beleuchtung, insbesondere in verschiedenen Farben, angesteuert werden können. Unter einer "Punktanzeige" soll in diesem Zusammenhang insbesondere eine Anzeige eines Anzeigemittels der Anzeigeeinheit verstanden werden, bei welchem eine Folge von Punkten, welche vorzugsweise in einer Linie angeordnet sind, zu einer Anzeige von Informationen dient. Vorzugsweise soll darunter insbesondere eine Anzeige verstanden werden, über welche Informationen mittels der Darstellung von Punkt der Punktanzeige angezeigt werden. Bevorzugt können die Punkte der Punktanzeige dazu einzeln angesteuert und beleuchtet werden. Besonders bevorzugt können die Punkte insbesondere in verschiedenen Farben angesteuert werden. Grundsätzlich wäre jedoch auch eine andere, einem Fachmann als sinnvoll erscheinende Ausbildung der abstrahierten Symbolanzeige denkbar, wie beispielsweise als Kreisanzeige, als Kreisabschnittsanzeige, wie insbesondere als Kreisdiagramm, als Linienanzeige oder dergleichen. Es sind insbesondere verschiedene Formen für die abstrahierte Symbolanzeige denkbar, wie beispielsweise Kreise, Quadrate, Rechtecke und/oder Linien. Dadurch kann insbesondere eine vorteilhaft abstrahierte Symbolanzeige bereitgestellt werden. Es kann insbesondere eine vorteilhaft einfache und leicht abzulesende abstrahierte Symbolanzeige bereitgestellt werden. Hierdurch kann insbesondere ein vorteilhaft hoher Bedienkomfort bereitgestellt werden.

Es wird weiter vorgeschlagen, dass die zumindest eine als Balken- oder Punktanzeige ausgebildete abstrahierte Symbolanzeige der Anzeigeeinheit, welche n diskrete Balkenpunkte oder Punkte besitzt, gemäß einer Skalierungsvorschrift zu einer Anzeige eines Werts eines Betriebs- und/oder Prozessparameters des externen Schweißgeräts als Balkenpunkt oder Punkt zwischen den Maximalwerten vorgesehen ist. Vorzugsweise wird ein Wert eines Betriebs- und/oder Prozessparameters des externen Schweißgeräts gemäß einer Skalierungsvorschrift, beispielsweise linear, exponentiell, polinomial und/oder logarithmisch auf einen skalierten Wert skaliert, sodass der skalierte Wert auf einer Skala, die n diskrete Balkenpunkte oder Punkte besitzt, als Balkenpunkte oder Punkte zwischen den Maximalwerten am Anfang und am Ende der Skala angezeigt werden kann. Insbesondere wird ein Wert durch eine Länge bzw. eine Menge von aufeinanderfolgenden Balkenpunkten oder Punkten der Balken- oder Punktanzeige dargestellt. Bevorzugt besitzt die Balken- oder Punktanzeige wenigstens 5, vorzugsweise wenigstens 10, bevorzugt wenigstens 15 und besonders bevorzugt wenigstens 20 diskrete Balkenpunkte oder Punkte. Dadurch kann insbesondere eine vorteilhaft leicht abzulesende Balken- oder Punktanzeige bereitgestellt werden. Es kann insbesondere eine vorteilhaft einfache und leicht zu interpretierende Balken- oder Punktanzeige bereitgestellt werden. Hierdurch kann insbesondere ein vorteilhaft hoher Bedienkomfort bereitgestellt werden.

Ferner wird vorgeschlagen, dass die zumindest eine als Balken- oder Punktanzeige ausgebildete abstrahierte Symbolanzeige der Anzeigeeinheit zumindest einen andersfarbigen Balkenpunkt oder Punkte aufweist, welcher als Orientierungshilfe dient. Vorzugsweise weist die Balken- oder Punktanzeige in regelmäßigen Abständen andersfarbige, insbesondere andersfarbig leuchtende, Balkenpunkte oder Punkte auf, welche als Orientierungshilfe und damit zu einem leichteren Ablesen der Balken- oder Punktanzeige dienen. Dadurch kann insbesondere eine vorteilhaft leicht abzulesende Balken- oder Punktanzeige bereitgestellt werden. Es kann insbesondere eine vorteilhaft einfache und leicht zu interpretierende Balken- oder Punktanzeige bereitgestellt werden. Vorzugsweise kann dadurch auch bei einem Verschwimmen der einzelnen Balkenpunkte oder Punkte der Balken- oder Punktanzeige ein genaues Ablesen der Balken- oder Punktanzeige ermöglicht werden.

Des Weiteren wird vorgeschlagen, dass die zumindest eine abstrahierte Symbolanzeige der Anzeigeeinheit dazu vorgesehen ist, einen zu einer Anzeige vorgesehenen Wert eines Betriebs- und/oder Prozessparameters des externen Schweißgeräts codiert darzustellen. Vorzugsweise wird die abstrahierte Symbolanzeige hierbei insbesondere zu einer Darstellung von Codesegmenten genutzt, die von einem Bediener interpretiert werden. Der Bediener muss die Codesegmente dabei insbesondere zu einem Wert eines Betriebs- und/oder Prozessparameters zusammensetzen. Hierdurch kann insbesondere eine vorteilhaft einfache Ablesbarkeit der abstrahierten Symbolanzeige erreicht werden. Insbesondere bei schwer zu unterscheidenden Werten und/oder bei der Anzeige von geringen Zahlenwerten kann durch eine Codierung ein Ablesen vorteilhaft vereinfacht werden.

Es wird ferner vorgeschlagen, dass die zumindest eine als Balken- oder Punktanzeige ausgebildete abstrahierte Symbolanzeige der Anzeigeeinheit dazu vorgesehen ist, einen zu einer Anzeige vorgesehenen Wert durch eine Folge von langen und/oder kurzen Balkensegmenten oder Punktfolgen darzustellen. Hierbei wäre beispielsweise denkbar, dass einem langen Balkensegment oder einer langen Punktfolge ein anderer Teilwert, wie beispielsweise der Wert 5, zugeordnet ist, als dem kurzen Balkensegment oder einer kurzen Punktfolge, welchem beispielsweise der Wert 1 zugeordnet werden könnte. Durch eine Folge von langen und/oder kurzen Balkensegmenten oder Punktfolgen kann dadurch ein Gesamtwert erzeugt werden. Alternativ könnte für den Wert 5 auch die Hälfte des Balkens oder der Punktanzeige ausgeleuchtet werden. Zu einem Ablesen müsste daher beispielsweise nun einfach eine Anzahl von langen und kurzen aufleuchtenden Balkensegmenten der Punktfolgen gezählt und zusammengerechnet werden. Dadurch kann insbesondere bei unscharfer Darstellung zuverlässig eine konkrete Nummer wahrgenommen werden. Es kann insbesondere eine vorteilhafte Ablesbarkeit von Zahlen erreicht werden.

Es wird weiter vorgeschlagen, dass die zumindest eine abstrahierte Symbolanzeige der Anzeigeeinheit dazu vorgesehen ist, einen Alarmzustand auszugeben. Vorzugsweise ist die zumindest eine abstrahierte Symbolanzeige der Anzeigeeinheit dazu vorgesehen, einen Alarmzustand des externen Schweißgeräts auszugeben. Hierdurch kann insbesondere eine vorteilhafte Nutzung der abstrahierten Symbolanzeige erreicht werden. Es kann insbesondere erreicht werden, dass ein Alarmzustand, insbesondere des externen Schweißgeräts, vorteilhaft schnell und zuverlässig von einem Bediener wahrgenommen werden kann. Es kann zuverlässig gewährleistetet werden, dass der Alarmzustand von dem Bediener wahrgenommen wird. Hierdurch kann eine hohe Betriebssicherheit gewährleistet werden.

Zudem wird vorgeschlagen, dass die zumindest eine abstrahierte Symbolanzeige der Anzeigeeinheit dazu vorgesehen ist, einen Alarmzustand durch die Ausgabe eines blinkenden Alarmmusters auszugeben. Vorzugsweise wird mittels des Alarmmusters eine Alarmnummer dargestellt. Das Alarmmuster kann dabei sowohl in der blinkenden abstrahierten Symbolanzeige ausgegeben werden als auch in einer separaten, abstrahierten Symbolanzeige. Verschiedene Alarme können durch das Alarmmuster insbesondere über Muster abgebildet werden. Hier sind beliebige Kombinationen von Farben und Positionen sowie Anzahl von Feldern anzudenken. Durch das Verwenden verschiedener Farben sind viele verschiedene Kombinationen möglich. Dadurch kann besonders zuverlässig ein Alarmzustand ausgegeben werden. Es kann besonders zuverlässig gewährleistetet werden, dass der Alarmzustand von dem Bediener wahrgenommen wird.

Ferner wird vorgeschlagen, dass die Schutzkassette zumindest eine Kommunikationseinheit zu einer Datenkommunikation mit dem externen Schweißgerät aufweist. Unter einer "Kommunikationseinheit" soll in diesem Zusammenhang insbesondere eine Einheit verstanden werden, welche zu einer Bereitstellung einer, insbesondere kabellosen, Kommunikation mit dem externen Schweißgerät vorgesehen ist. Vorzugsweise weist die Kommunikationseinheit zu einer Kommunikation mit dem externen Schweißgerät zumindest eine Schnittstelle auf. Vorzugsweise soll unter einer Kommunikationseinheit insbesondere eine Einheit verstanden werden, welche zu einem Austausch von Daten vorgesehen ist. Insbesondere weist die Kommunikationseinheit zumindest einen Informationseingang und zumindest einen Informationsausgang auf. Vorzugsweise weist die Kommunikationseinheit zumindest zwei Informationseingänge und zumindest zwei Informationsausgänge auf, wobei jeweils zumindest ein Informationseingang und zumindest ein Informationsausgang zu einer Verbindung mit einem physischen System, insbesondere dem externen Schweißgerät, vorgesehen sind. Besonders bevorzugt soll darunter eine Schnittstelle zwischen zumindest zwei physischen Systemen, wie insbesondere zwischen dem externen Schweißgerät und der Schutzkassette, verstanden werden. Es sind verschiedene, dem Fachmann als sinnvoll erscheinende Kommunikationseinheiten denkbar, insbesondere soll darunter jedoch eine drahtlose Schnittstelle, wie beispielsweise Bluetooth, WLAN, Zigbee, NFC, RFID, GSM, LTE oder UMTS, und/oder eine drahtgebundene Schnittstelle, wie beispielsweise ein USB-Anschluss, eine Canbus-Schnittstelle, eine RS485 Schnittstelle, eine Ethernet-Schnittstelle, eine optische Schnittstelle, eine KNX-Schnittstelle und/oder eine Powerline-Schnittstelle, verstanden werden. Dadurch können vorteilhaft zuverlässig Daten, insbesondere Betriebs und/oder Prozessparameter, des externen Schweißgeräts empfangen und/oder abgerufen werden.

Die erfindungsgemäße Schutzkassette, die Blendschutzvorrichtung, das System sowie das Verfahren sollen hierbei nicht auf die oben beschriebene Anwendung und Ausführungsform beschränkt sein. Insbesondere können die erfindungsgemäße Schutzkassette, die Blendschutzvorrichtung, das System sowie das Verfahren zu einer Erfüllung einer hierin beschriebenen Funktionsweise eine von einer hierin genannten Anzahl von einzelnen Elementen, Bauteilen und Einheiten abweichende Anzahl aufweisen.

### Zeichnungen

Weitere Vorteile ergeben sich aus der folgenden Zeichnungsbeschreibung. In den Zeichnungen sind zwei Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine Blendschutzvorrichtung mit einer erfindungsgemäßen Schutzkassette und ein externes Schweißgerät in einer schematischen Darstellung,
- Fig. 2: die erfindungsgemäße Schutzkassette mit einem optischen Blendschutzfilter und mit einer Anzeigeeinheit, welche eine erste abstrahierte Symbolanzeige und eine zweite abstrahierte Symbolanzeige aufweist, in einer schematischen Darstellung,
- Fig. 3A: die erste abstrahierte Symbolanzeige während der Darstellung eines ersten Werts in einer schematischen Darstellung,
- Fig. 3B: die erste abstrahierte Symbolanzeige während der Darstellung eines zweiten Werts in einer schematischen Darstellung,
- Fig. 3C: die erste abstrahierte Symbolanzeige während der Darstellung eines dritten Werts in einer schematischen Darstellung,
- Fig. 3D: die erste abstrahierte Symbolanzeige während der Darstellung eines vierten Werts in einer schematischen Darstellung,
- Fig. 3E: die erste abstrahierte Symbolanzeige während der Darstellung eines fünften Werts in einer schematischen Darstellung,
- Fig. 3F: die erste abstrahierte Symbolanzeige während der Darstellung eines sechsten Werts in einer schematischen Darstellung,
- Fig. 3G: die erste abstrahierte Symbolanzeige während der Darstellung eines siebten Werts in einer schematischen Darstellung,
- Fig. 4A: die zweite abstrahierte Symbolanzeige während der Darstellung eines ersten Werts in einer schematischen Darstellung,
- Fig. 4B: die zweite abstrahierte Symbolanzeige während der Darstellung eines zweiten Werts in einer schematischen Darstellung,
- Fig. 4C: die zweite abstrahierte Symbolanzeige während der Darstellung eines dritten Werts in einer schematischen Darstellung,
- Fig. 4D: die zweite abstrahierte Symbolanzeige während der Darstellung eines vierten Werts in einer schematischen Darstellung,
- Fig. 4E: die zweite abstrahierte Symbolanzeige während der Darstellung eines fünften Werts in einer schematischen Darstellung,
- Fig. 5A: die zweite abstrahierte Symbolanzeige während der Darstellung eines ersten Alarmmusters in einer schematischen Darstellung,
- Fig. 5B: die zweite abstrahierte Symbolanzeige während der Darstellung eines zweiten Alarmmusters in einer schematischen Darstellung,
- Fig. 5C: die zweite abstrahierte Symbolanzeige während der Darstellung eines dritten Alarmmusters in einer schematischen Darstellung,
- Fig. 5D: die zweite abstrahierte Symbolanzeige während der Darstellung eines vierten Alarmmusters in einer schematischen Darstellung,
- Fig. 5E: die zweite abstrahierte Symbolanzeige während der Darstellung eines fünften Alarmmusters in einer schematischen Darstellung,
- Fig. 6: ein schematischen Ablaufdiagramm eines Verfahrens zum Betrieb der erfindungsgemäßen Schutzkassette und
- Fig. 7: eine Blendschutzvorrichtung mit einer alternativen erfindungsgemäßen Schutzkassette in einer schematischen Schnittdarstellung.

### Beschreibung der Ausführungsbeispiele

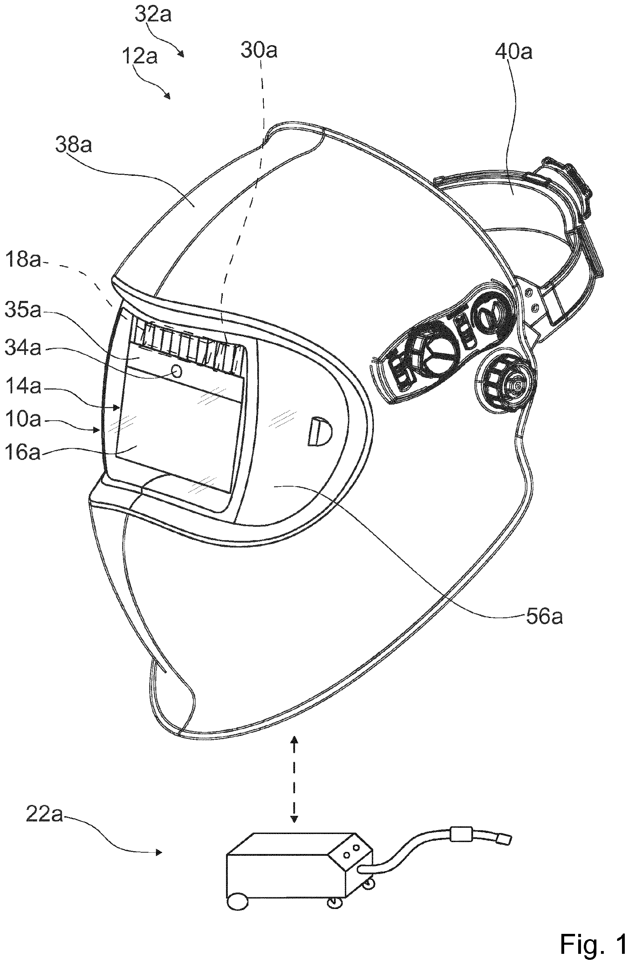

Die Figur 1 zeigt eine Blendschutzvorrichtung 12a. Die Blendschutzvorrichtung 12a ist dazu vorgesehen, während eines Betriebs von einem Bediener auf dem Kopf getragen zu werden. Der Bediener ist hierbei von einem Träger gebildet. Figur 1 zeigt die Blendschutzvorrichtung 12a in einer Betriebsstellung. Die Blendschutzvorrichtung 12a ist von einem Schweißhelm gebildet. Grundsätzlich wäre jedoch auch eine andere, einem Fachmann als sinnvoll erscheinende Ausbildung der Blendschutzvorrichtung 12a denkbar.

Die Blendschutzvorrichtung 12a weist eine Schutzkassette 10a auf. Die Schutzkassette 10a weist einen optischen Blendschutzfilter 14a auf. Der optische Blendschutzfilter 14a weist eine Flüssigkristallzelle 16a auf. Ferner weist die Schutzkassette 10a eine Steuer- und Regeleinheit 18a auf. Die Steuer- und Regeleinheit 18a ist dazu vorgesehen, abhängig von einem erfassten Arbeitszustand und einer Lichteinstrahlung eine Durchlässigkeit des optischen Blendschutzfilters 14a zu steuern. Die Steuer- und Regeleinheit 18a ist hierzu mit einer Sensoreinheit 34a verbunden, welche zu einer Erfassung eines Arbeitszustands und einer Lichteinstrahlung vorgesehen ist. Die Schutzkassette 10a weist die Sensoreinheit 34a auf. Die Sensoreinheit 34a weist zumindest einen Sensor auf, der dazu vorgesehen ist, einen Schweißvorgang oder das Auftreten eines hellen Lichts, welches die Augen eines Benutzers schädigen oder auf andere Weise beeinflussen könnte, zu detektieren. Der Sensor der Sensoreinheit 34a ist von einer Fotozelle gebildet. Grundsätzlich wäre jedoch auch eine andere, einem Fachmann als sinnvoll erscheinende Ausbildung des Sensors der Sensoreinheit 34a denkbar. Die Steuer- und Regeleinheit 18a ist dazu vorgesehen, die Daten der Sensoreinheit 34a zu verarbeiten und abhängig davon den optischen Blendschutzfilter 14a anzusteuern.

Der optische Blendschutzfilter 14a ist von einem elektrooptischen Filter gebildet. Der optische Blendschutzfilter 14a ist von einem automatic darkening filter,kurz ADF, gebildet. Der optische Blendschutzfilter 14a besteht aus mehreren Schichten. Der optische Blendschutzfilter 14a ist als ein Mehrschichtverbund ausgebildet. Eine Anzahl von Schichten ist hierbei lediglich beispielhaft und kann grundsätzlich variieren. Der optische Blendschutzfilter 14a weist eine Flüssigkristallschicht auf, welche als eine Blende des Blendschutzfilters 14a dient. Die Flüssigkristallschicht ist von einem transluzenten Flüssigkristallbildschirm gebildet. Die Flüssigkristallschicht wird von der Steuer- und Regeleinheit 18a angesteuert. Die Flüssigkristallschicht des optischen Blendschutzfilters 14a wird von der Steuer- und Regeleinheit 18a aktiviert, wenn über die Sensoreinheit 34a ein Schweißverfahren oder ein Lichtblitz erfasst wird. Die Flüssigkristallschicht bzw. die Flüssigkristallzelle 16a des Blendschutzfilters 14a wird von der Steuer- und Regeleinheit 18a abgedunkelt, wenn über die Sensoreinheit 34a ein Schweißverfahren oder ein Lichtblitz erfasst wird. Der optische Blendschutzfilter 14a weist eine rechteckige Grundform auf.

Die Blendschutzvorrichtung 12a weist eine Schildeinheit 38a auf. Die Schutzkassette 10a ist fest in der Schildeinheit 38a aufgenommen. Die Schutzkassette 10a ist positionsfest in der Schildeinheit 38a aufgenommen. Die Schutzkassette 10a ist in eine Ausnehmung in der Schildeinheit 38a eingepasst. Grundsätzlich wäre jedoch auch denkbar, dass die Schutzkassette 10a in die Schildeinheit 38a integriert ist. Die Schildeinheit 38a besteht aus einem im Wesentlichen formfesten Material. Die Schildeinheit 38a besteht aus einem Kunststoff, welcher insbesondere beständig gegenüber Funken und/oder anderen, beim Schweißen auftretenden Einflüssen ist. Die Schildeinheit 38a ist dazu vorgesehen, insbesondere nach den einschlägigen Normen für Schweißmasken, ein Gesicht und/oder Kopf des Bedieners zu bedecken und zu schützen. Die Schildeinheit 38a weist eine teilweise einer Kopfform angepasste Form auf. Die Schildeinheit 38a ist in einem getragenen Zustand der Blendschutzvorrichtung 12a teilweise um ein Gesicht des Bedieners gebogen. Des Weiteren weist die Blendschutzvorrichtung 12a eine Kopfbefestigungseinheit 40a auf. Die Kopfbefestigungseinheit 40a ist zu einer Befestigung an dem Kopf des Bedieners vorgesehen. Die Kopfbefestigungseinheit 40a ist von einem Kopfband gebildet. Die Kopfbefestigungseinheit 40a ist nicht weiter sichtbar mit der Schildeinheit 38a verbunden.

Die Blendschutzvorrichtung 12a weist ferner eine Vorsatzscheibe 56a auf. Die Vorsatzscheibe 56a ist mit der Schildeinheit 38a über nicht weiter sichtbare Rastelemente verbunden. Vorzugsweise weist die Vorsatzscheibe 56a zwei gegenüberliegende Rastausnehmungen auf, in welche jeweils ein Rastelement der Schildeinheit 38a rastend eingreift. Durch die Verrastung kann die Vorsatzscheibe 56a einfach demontiert werden. Hierdurch sind eine einfache Reinigung und/oder ein einfacher Austausch möglich. Die Vorsatzscheibe 56a ist transparent ausgebildet. Die Vorsatzscheibe 56a ist zu einem Schutz der Schutzkassette 10a vorgesehen. Die Vorsatzscheibe 56a bildet ein Teil der Schutzkassette 10a. Die Vorsatzscheibe 56a verdeckt den optischen Blendschutzfilter 14a der Schutzkassette 10a von außen. Hierdurch kann eine Beschädigung des optischen Blendschutzfilters 14a der Schutzkassette 10a vermieden werden. Bei einer Beschädigung der Vorsatzscheibe 56a der Schutzkassette 10a kann diese, insbesondere im Gegensatz zu dem optischen Blendschutzfilter 14a der Schutzkassette 10a, einfach und kostengünstig ausgetauscht werden.

Die Schutzkassette 10a weist ein Gehäuse 35a auf, in welchem der optische Blendschutzfilter 14a teilweise aufgenommen ist. Die Steuer- und Regeleinheit 18a und die Sensoreinheit 34a sind ebenfalls in dem Gehäuse 35a der Schutzkassette 10a aufgenommen. Die Sensoreinheit 34a ist, während eines Betriebs der Blendschutzvorrichtung 12a, teilweise auf einer einem Gesicht des Bedieners abgewandten Außenseite der Schutzkassette 10a angeordnet.

Des Weiteren weist die Schutzkassette 10a eine Kommunikationseinheit 30a zu einer Datenkommunikation mit einem externen Schweißgerät 22a auf. Die Kommunikationseinheit 30a ist zu einer drahtlosen Datenkommunikation mit dem externen Schweißgerät 22a vorgesehen. Die Kommunikationseinheit 30a weist eine Funkschnittstelle zu einer drahtlosen Datenkommunikation mit dem externen Schweißgerät 22a auf. Die Funkschnittstelle der Kommunikationseinheit 30a ist von einer Bluetooth-Schnittstelle gebildet. Grundsätzlich wäre jedoch auch eine andere, einem Fachmann als sinnvoll erscheinende Ausbildung der Funkschnittstelle und/oder der Kommunikationseinheit 30a denkbar. Über die Kommunikationseinheit 30a werden Betriebs- und Prozessparameter des externen Schweißgeräts 22a von dem externen Schweißgerät 22a auf die Blendschutzvorrichtung 12a übertragen. Die Betriebs und Prozessparameter des externen Schweißgeräts 22a werden über die Kommunikationseinheit 30a auf die Schutzkassette 10a übertragen.

Das externe Schweißgerät 22a verfügt ebenfalls über eine nicht weiter sichtbare Kommunikationseinheit zu einer Datenkommunikation mit der Kommunikationseinheit 30a der Schutzkassette 10a. Das externe Schweißgerät 22a und die Blendschutzvorrichtung 12a mit der Schutzkassette 10a bilden ein System 32a.

Ferner weist die Schutzkassette 10a eine Anzeigeeinheit 20a auf. Die Anzeigeeinheit 20a ist zu einer Anzeige von Betriebs- und Prozessparametern des externen Schweißgeräts 22a vorgesehen. Die Anzeigeeinheit 20a weist zumindest eine abstrahierte Symbolanzeige 24a, 26a auf. Die Anzeigeeinheit 20a weist mehrere abstrahierte Symbolanzeigen 24a, 26a, 52a, 54a auf. Die abstrahierten Symbolanzeigen 24a, 26a, 52a, 54a sind jeweils zu einer Anzeige unterschiedlicher Betriebs- und Prozessparameter des externen Schweißgeräts 22a vorgesehen. Die abstrahierten Symbolanzeigen 24a, 26a, 52a, 54a sind zumindest während eines Betriebs in einem unfokussierbaren Sichtfeld des Bedieners angeordnet. Die abstrahierten Symbolanzeigen 24a, 26a, 52a, 54a der Anzeigeeinheit 20a sind während eines Betriebs in einem peripheren Sichtfeld des Bedieners angeordnet. Die abstrahierten Symbolanzeigen 24a, 26a, 52a, 54a der Anzeigeeinheit 20a sind während eines Betriebs, zumindest bei gerader Kopfhaltung und einem direkten Geradeausschauen des Bedieners in einem peripheren Sichtfeld angeordnet. Die abstrahierten Symbolanzeigen 24a, 26a, 52a, 54a sind auf einer einem Gesicht des Bedieners zugewandten Innenseite der Schutzkassette 10a angeordnet. Die abstrahierten Symbolanzeigen 24a, 26a, 52a, 54a sind beispielspielhaft auf allen vier Seiten der Flüssigkristallzelle 16a des optischen Blendschutzfilters 14a angeordnet. Grundsätzlich wäre jedoch auch eine andere, einem Fachmann als sinnvoll erscheinende Anordnung der abstrahierten Symbolanzeigen 24a, 26a, 52a, 54a denkbar. Die abstrahierten Symbolanzeigen 24a, 26a, 52a, 54a können grundsätzlich beliebig um den optischen Blendschutzfilter 14a angeordnet werden. Aufgrund der Anordnung der Schutzkassette 10a während eines Betriebs dicht vor einem Gesicht des Bedieners, kann ein Bediener nicht oder nur schwer auf die abstrahierten Symbolanzeigen 24a, 26a, 52a, 54a fokussieren. Blickt der Bediener ferner durch den optischen Blendschutzfilter 14a hindurch auf ein entferntes Objekt, muss zu einer Betrachtung einer Anzeige auf der Schutzkassette 10a ständig neu fokussiert werden, was auf Dauer anstrengend sein kann und zu Müdigkeit führen kann. Mittels der abstrahierten Symbolanzeigen 24a, 26a, 52a, 54a kann auch ohne ein Fokussieren auf die abstrahierten Symbolanzeigen 24a, 26a, 52a, 54a sinnvoll eine Information abgelesen werden. Dadurch kann der Bediener und dessen Augen geschont werden.

Zwei der abstrahierten Symbolanzeigen 24a, 26a der Anzeigeeinheit 20a sind jeweils von einer Balkenanzeige gebildet. Die zwei abstrahierten Symbolanzeigen 24a, 26a der Anzeigeeinheit 20a sind jeweils von einer Balkenanzeige gebildet, welche n diskrete Balkenpunkte 28a, 36a besitzen. Die abstrahierten Symbolanzeigen 24a, 26a der Anzeigeeinheit 20a besitzen jeweils beispielhaft vierundzwanzig diskrete Balkenpunkte 28a, 36a. Die Balkenpunkte 28a, 36a der abstrahierten Symbolanzeigen 24a, 26a sind jeweils voneinander getrennt. Ferner sind die Balkenpunkte 28a, 36a der abstrahierten Symbolanzeigen 24a, 26a jeweils in zwei verschiedenen Farben beleuchtbar. Die Balkenpunkte 28a, 36a weisen daher drei mögliche Zustände auf: einen unbeleuchteten Zustand, einen Zustand, bei welchem der Balkenpunkt 28a, 36a in einer ersten Farbe beleuchtet wird, und einen Zustand, bei welchem der Balkenpunkt 28a, 36a in einer zweiten Farbe beleuchtet wird. Die erste Farbe ist hierbei beispielhaft rot, während die zweite Farbe beispielhaft grün ist. Die verschiedenen Farben der Balkenpunkte 28a, 36a sind jeweils durch unterschiedliche Schraffuren dargestellt. Weist ein Balkenpunkt 28a, 36a keine Schraffur auf, ist er unbeleuchtet. Die Schraffur von unten links nach oben rechts steht dabei beispielhaft für die erste Farbe. Die Schraffur von oben links nach unten rechts steht dabei beispielhaft für die zweite Farbe. Grundsätzlich wäre jedoch auch eine andere, einem Fachmann als sinnvoll erscheinende Ausgestaltung der abstrahierten Symbolanzeigen 24a, 26a denkbar, wie beispielsweise als kontinuierliche Balkenanzeige oder als Kreisdiagramm. Es wäre auch denkbar, dass die Balkenpunkte 28a, 36a der abstrahierten Symbolanzeigen 24a, 26a lediglich in einer Farbe beleuchtbar sind.

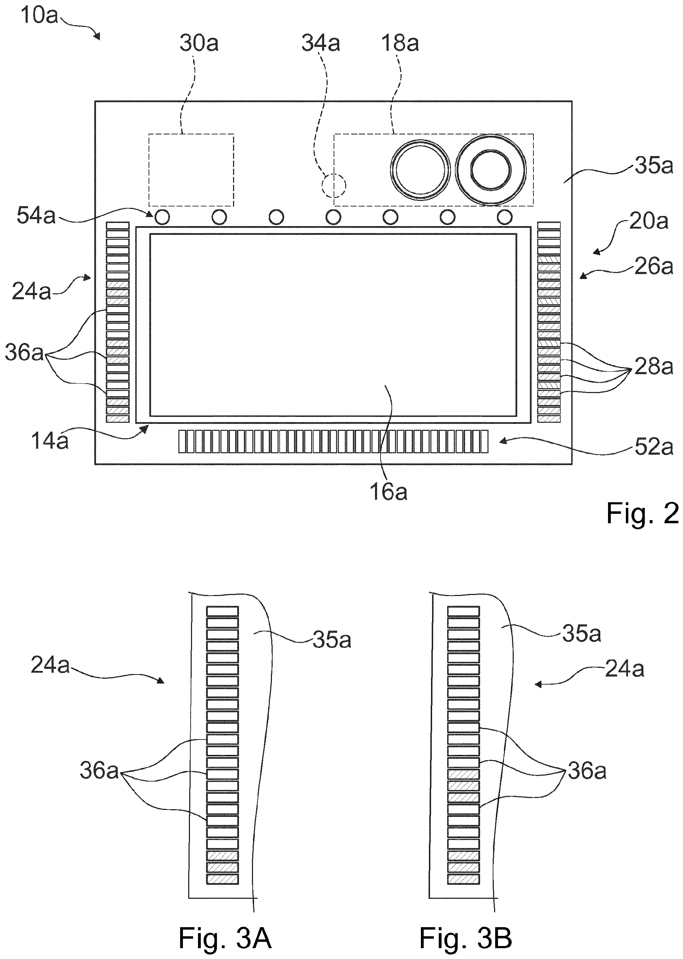

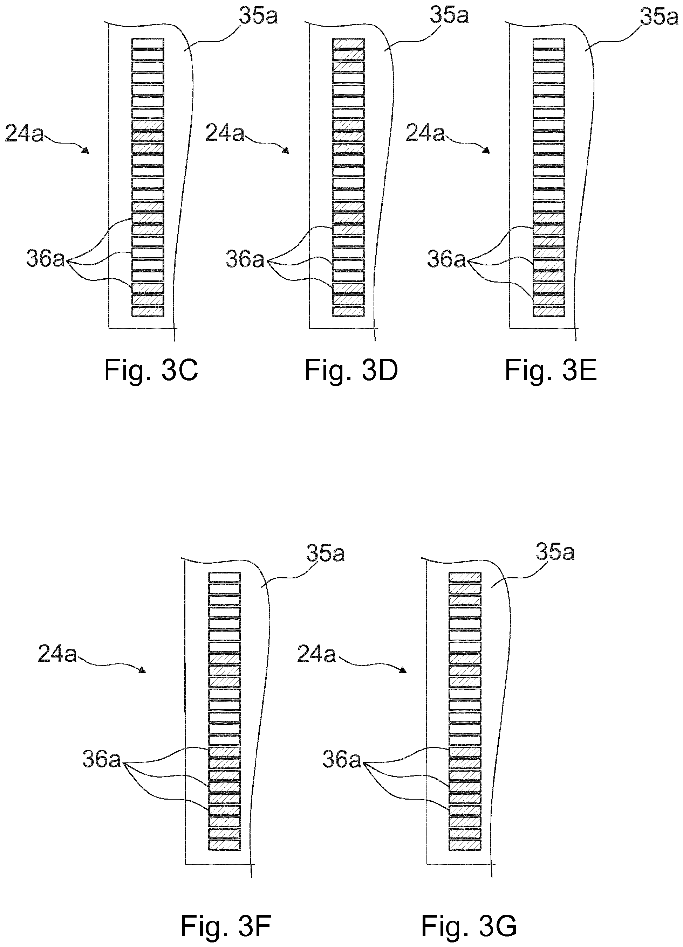

Die erste abstrahierte Symbolanzeige 24a der Anzeigeeinheit 20a ist zu einer Anzeige einer aktuell eingestellten Programmnummer des externen Schweißgeräts 22a vorgesehen. Grundsätzlich wären jedoch auch andere Betriebs- und Prozessparameter des externen Schweißgeräts 22a zu einer Anzeige denkbar, wie beispielsweise ein Strom, eine Spannung, ein Drahtvorschub, ein Gasfluss, eine Temperatur, ein Arbeitsfortschritt, ein Zustand und/oder ein Prozess des externen Schweißgeräts 22a. Die erste abstrahierte Symbolanzeige 24a der Anzeigeeinheit 20a ist dazu vorgesehen, einen zu einer Anzeige vorgesehenen Wert eines Betriebs- oder Prozessparameters des externen Schweißgeräts 22a codiert darzustellen. Die erste abstrahierte Symbolanzeige 24a der Anzeigeeinheit 20a ist dazu vorgesehen, eine zu einer Anzeige vorgesehene aktuell eingestellte Programmnummer des externen Schweißgeräts 22a codiert darzustellen. Die als Balkenanzeige ausgebildete erste abstrahierte Symbolanzeige 24a der Anzeigeeinheit 20a ist dazu vorgesehen, einen zu einer Anzeige vorgesehenen Wert durch eine Folge von langen und kurzen Balkensegmenten darzustellen. Die erste abstrahierte Symbolanzeige 24a der Anzeigeeinheit 20a ist dazu vorgesehen, eine zu einer Anzeige vorgesehene Programmnummer durch eine Folge von langen und kurzen Balkensegmenten darzustellen. Hierbei ist einem langen beleuchteten Balkensegment ein anderer Teilwert zugeordnet, als einem kurzen beleuchteten Balkensegment. Einem langen beleuchteten Balkensegment ist beispielhaft der Zahlenwert fünf zugeordnet. Ein langes beleuchtetes Balkensegment besteht ferner beispielhaft aus neun aufeinanderfolgenden Balkenpunkten 36a. Einem kurzen beleuchteten Balkensegment ist dagegen beispielhaft der Zahlenwert eins zugeordnet. Ein kurzes beleuchtetes Balkensegment besteht ferner beispielhaft aus drei aufeinanderfolgenden Balkenpunkten 36a. Zu einem Ablesen der ersten abstrahierten Symbolanzeige 24a kann ein Bediener nun die Anzahl kurzer Balkensegmente mal eins mit der Anzahl langer Balkensegmente mal fünf addieren. Der herauskommende Zahlenwert entspricht einer aktuell eingestellten Programmnummer des externen Schweißgeräts 22a. Verschiedene externe Schweißgeräte 22a können dabei insbesondere mit verschiedenen Farben dargestellt werden, um bei einer Verbindung mit mehreren Schweißgeräten 22a die Programmnummer eindeutig zuordnen zu können. Die Figuren 3A bis 3G zeigen die erste abstrahierte Symbolanzeige 24a mit verschieden dargestellten Programmnummern. In der Figur 3A ist in der ersten abstrahierten Symbolanzeige 24a ein kurzes Balkensegment dargestellt, was der Programmnummer 1 entspricht. In der Figur 3B sind in der ersten abstrahierten Symbolanzeige 24a zwei kurze Balkensegmente dargestellt, was der Programmnummer 2 entspricht. In der Figur 3C sind in der ersten abstrahierten Symbolanzeige 24a drei kurze Balkensegmente dargestellt, was der Programmnummer 3 entspricht. In der Figur 3D sind in der ersten abstrahierten Symbolanzeige 24a vier kurze Balkensegmente dargestellt, was der Programmnummer 4 entspricht. In der Figur 3E ist in der ersten abstrahierten Symbolanzeige 24a ein langes Balkensegment dargestellt, was der Programmnummer 5 entspricht. In der Figur 3F sind in der ersten abstrahierten Symbolanzeige 24a ein kurzes Balkensegment und ein langes Balkensegment dargestellt, was der Programmnummer 6 entspricht. In der Figur 3G sind in der ersten abstrahierten Symbolanzeige 24a zwei kurze Balkensegmente und ein langes Balkensegment dargestellt, was der Programmnummer 7 entspricht.

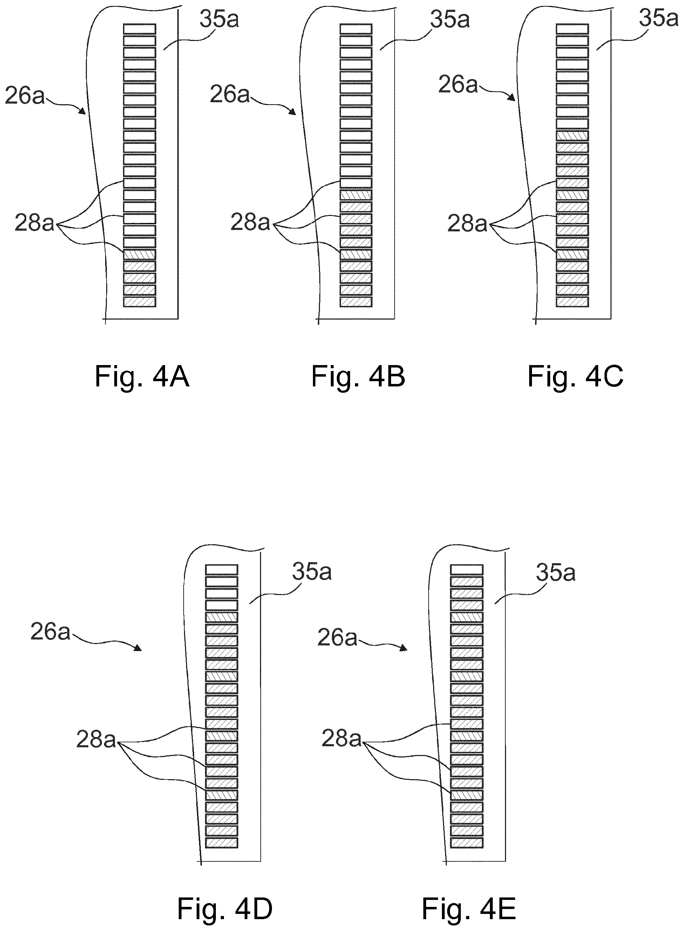

Die zweite abstrahierte Symbolanzeige 26a der Anzeigeeinheit 20a ist zu einer Anzeige einer aktuellen Stromstärke des externen Schweißgeräts 22a vorgesehen. Grundsätzlich wären jedoch auch andere Betriebs- und Prozessparameter des externen Schweißgeräts 22a zu einer Anzeige denkbar. Die als Balkenanzeige ausgebildete zweite abstrahierte Symbolanzeige 26a der Anzeigeeinheit 20a, welche n diskrete Balkenpunkte 28a besitzt, ist gemäß einer Skalierungsvorschrift zu einer Anzeige eines Werts eines Betriebs- und Prozessparameters des externen Schweißgeräts 22a als Balkenpunkt 28a zwischen den Maximalwerten vorgesehen ist. Die zweite abstrahierte Symbolanzeige 26a der Anzeigeeinheit 20a ist dazu vorgesehen, einen Wert einer aktuellen Stromstärke des externen Schweißgeräts 22a gemäß einer Skalierungsvorschrift als mehrere, von einem Nullpunkt ausgehende, fortlaufende Balkenpunkte 28a darzustellen. Die zweite abstrahierte Symbolanzeige 26a der Anzeigeeinheit 20a ist daher dazu vorgesehen, einen Wert einer aktuellen Stromstärke des externen Schweißgeräts 22a gemäß einer Skalierungsvorschrift als eine Länge bzw. eine Menge von aufeinanderfolgenden Balkenpunkten 28a der abstrahierten Symbolanzeige 26a darzustellen. Jeder Balkenpunkt 28a der abstrahierten Symbolanzeige 26a ist einem Wert von 10 A gleichgesetzt, sodass über eine Anzahl beleuchteter Balkenpunkte 28a auf eine aktuelle Stromstärke des externen Schweißgeräts 22a geschlossen werden kann. Die als Balkenanzeige ausgebildete abstrahierte Symbolanzeige 26a der Anzeigeeinheit 20a weist mehrere andersfarbige Balkenpunkte 28a auf, welche als Orientierungshilfe dienen. Die als Balkenanzeige ausgebildete abstrahierte Symbolanzeige 26a der Anzeigeeinheit 20a steuert in gleichmäßigen Abständen Balkenpunkte 28a in der zweiten Farbe an. Bei der zweiten abstrahierten Symbolanzeige 26a wird beispielhaft jeder fünfte beleuchtete Balkenpunkt 28a in der zweiten Farbe angesteuert. Hierdurch kann eine leichte Ablesbarkeit erreicht werden. Insbesondere kann der Wert der aktuellen Stromstärke des externen Schweißgeräts 22a vorteilhaft auch über die Balkenpunkte 28a, welche in der zweiten Farbe angesteuert werden, abgeschätzt werden. Die Figuren 4A bis 3E zeigen die zweite abstrahierte Symbolanzeige 26a mit verschieden dargestellten Stromstärken. In der Figur 4A sind in der zweiten abstrahierten Symbolanzeige 24a fortlaufend von unten fünf Balkenpunkte 28a beleuchtet, was einer Stromstärke von 50 A entspricht. Der fünfte Balkenpunkt 28a von unten ist als Orientierungshilfe in der zweiten Farbe beleuchtet. In der Figur 4B sind in der zweiten abstrahierten Symbolanzeige 24a fortlaufend von unten zehn Balkenpunkte 28a beleuchtet, was einer Stromstärke von 100 A entspricht. Der fünfte und zehnte Balkenpunkt 28a von unten sind als Orientierungshilfen in der zweiten Farbe beleuchtet. In der Figur 4C sind in der zweiten abstrahierten Symbolanzeige 24a fortlaufend von unten fünfzehn Balkenpunkte 28a beleuchtet, was einer Stromstärke von 150 A entspricht. Der fünfte, der zehnte und der fünfzehnte Balkenpunkt 28a von unten sind als Orientierungshilfen in der zweiten Farbe beleuchtet. In der Figur 4D sind in der zweiten abstrahierten Symbolanzeige 24a fortlaufend von unten zwanzig Balkenpunkte 28a beleuchtet, was einer Stromstärke von 200 A entspricht. Der fünfte, der zehnte, der fünfzehnte und der zwanzigste Balkenpunkt 28a von unten sind als Orientierungshilfen in der zweiten Farbe beleuchtet. In der Figur 4E sind in der zweiten abstrahierten Symbolanzeige 24a fortlaufend von unten dreiundzwanzig Balkenpunkte 28a beleuchtet, was einer Stromstärke von 230 A entspricht. Der fünfte, der zehnte, der fünfzehnte und der zwanzigste Balkenpunkt 28a von unten sind als Orientierungshilfen in der zweiten Farbe beleuchtet.

Eine dritte abstrahierte Symbolanzeige 52a der Anzeigeeinheit 20a ist ebenfalls von einer Balkenanzeige gebildet. Die dritte abstrahierte Symbolanzeige 52a der Anzeigeeinheit 20a ist von einer Balkenanzeige gebildet, welche n diskrete Balkenpunkte besitzt. Über die dritte abstrahierte Symbolanzeige 52a können weitere, einem Fachmann als erscheinende Betriebs- und Prozessparameter des externen Schweißgeräts 22a ausgegeben werden. Es wäre beispielsweise denkbar die dritte abstrahierte Symbolanzeige 52a als Schweisskorrekturhilfe zu verwenden, bei der der Prozess mittels der Balkenanzeige als gut oder schlecht bewertet wird und mittels einem leuchtenden Balkenpunkt zwischen zwei Grenzen angezeigt wird. Die Grenzen eines guten Prozesses können durch zwei weitere Balkenpunkte dargestellt werden, welche in einer zweiten Farbe leuchten. Falls der Prozess gut ist, bleibt der leuchtende Balkenpunkt in der Mitte. Verschiebt sich der leuchtende Balkenpunkt zu einem Extremwert, kann dies durch Anpassen des Prozesses wieder korrigiert werden, bis der leuchtende Balkenpunkt wieder in die Mitte wandert. So lässt sich ein einfaches Feedbacksystem einrichten.

Eine vierte abstrahierte Symbolanzeige 54a der Anzeigeeinheit 20a ist von einer Punktanzeige gebildet. Die dritte abstrahierte Symbolanzeige 54a der Anzeigeeinheit 20a ist von einer Punktanzeige gebildet, welche n diskrete Punkte besitzt. Über die vierte abstrahierte Symbolanzeige 54a können weitere, einem Fachmann als sinnvoll erscheinende Betriebs- und Prozessparameter des externen Schweißgeräts 22a ausgegeben werden. Über die vierte abstrahierte Symbolanzeige 54a könnten beispielsweise optionale Programme angezeigt werden.

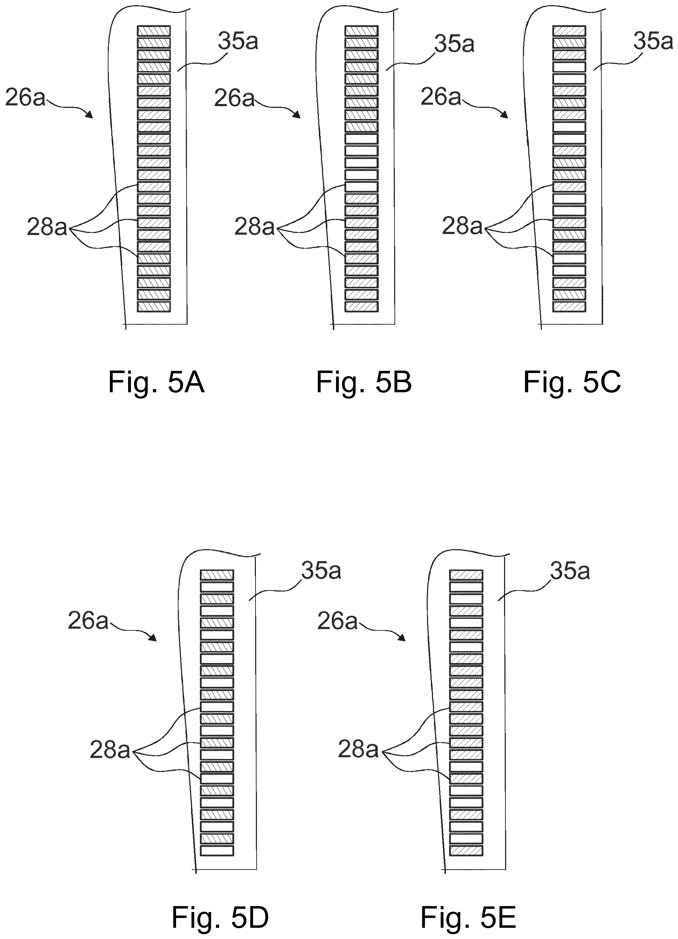

Ferner sind die abstrahierten Symbolanzeigen 24a, 26a der Anzeigeeinheit 20a dazu vorgesehen, einen Alarmzustand auszugeben. Die abstrahierten Symbolanzeigen 24a, 26a der Anzeigeeinheit 20a sind dazu vorgesehen, einen Alarmzustand des externen Schweißgeräts 22a auszugeben. Dabei können verschiedene Alarmzustände des externen Schweißgeräts 22a ausgegeben werden. Es können verschiedene, einem Fachmann als sinnvoll erscheinende Alarmzustände des externen Schweißgeräts 22a ausgegeben werden. Die abstrahierten Symbolanzeigen 24a, 26a der Anzeigeeinheit 20a sind dazu vorgesehen, einen Alarmzustand durch die Ausgabe eines blinkenden Alarmmusters auszugeben. Mittels des Alarmmusters wird dabei eine Alarmnummer dargestellt. Vorzugsweise blinken in einem Alarmzustand beide abstrahierten Symbolanzeigen 24a, 26a, wobei über die erste abstrahierte Symbolanzeige 24a lediglich die Information ausgegeben wird, dass eine Fehlermeldung vorliegt und über die zweite erste abstrahierte Symbolanzeige 26a das Alarmmuster ausgeben wird, welches einer Alarmnummer gleichzusetzen ist. Grundsätzlich wäre jedoch auch denkbar, dass die Alarmnummer mittels der bereits beschriebenen Codierung der ersten abstrahierten Symbolanzeige 24a angezeigt wird. Für die Alarmmuster sind beliebige Kombinationen von Farben und Positionen oder Anzahl von Feldern etc. denkbar, welche eindeutig voneinander unterscheidbar sind. Durch das Hinzufügen weiterer Farben wären grundsätzlich viele verschiedene weitere Kombinationen möglich. Die Figuren 5A bis 5E zeigen die zweite abstrahierte Symbolanzeige 26a mit verschiedenen Alarmmustern, welche jeweils für verschiedene Alarmzustände stehen. In der Figur 5A ist in der zweiten abstrahierten Symbolanzeige 24a ein langes Balkensegment in der Mitte in der ersten Farbe beleuchtet, an welches auf beiden Seiten Balkensegmente in der zweiten Farbe angrenzen. In der Figur 5B ist in der zweiten abstrahierten Symbolanzeige 24a von unten her ein langes Balkensegment in der ersten Farbe beleuchtet und von oben her ein langes Balkensegment in der zweiten Farbe beleuchtet. In der Figur 5C sind in der zweiten abstrahierten Symbolanzeige 24a mehrere getrennt voneinander angeordnete Balkensegmente beleuchtet, die jeweils aus einem Balkenpunkt 28a in der zweiten Farbe und zwei Balkenpunkten 28a in der zweiten Farbe bestehen, die jeweils an den Balkenpunkt 28a in der zweiten Farbe angrenzen. In der Figur 5D ist in der zweiten abstrahierten Symbolanzeige 24a jeweils abwechselnd ein Balkenpunkt 28a beleuchtet und ein Balkenpunkt 28a unbeleuchtet. In der Figur 5D ist in der zweiten abstrahierten Symbolanzeige 24a ein langes Balkensegment in der Mitte beleuchtet, während weitere einzelne Balkenpunkte 28a oberhalb und unterhalb des langen Balkensegments beleuchtet sind.

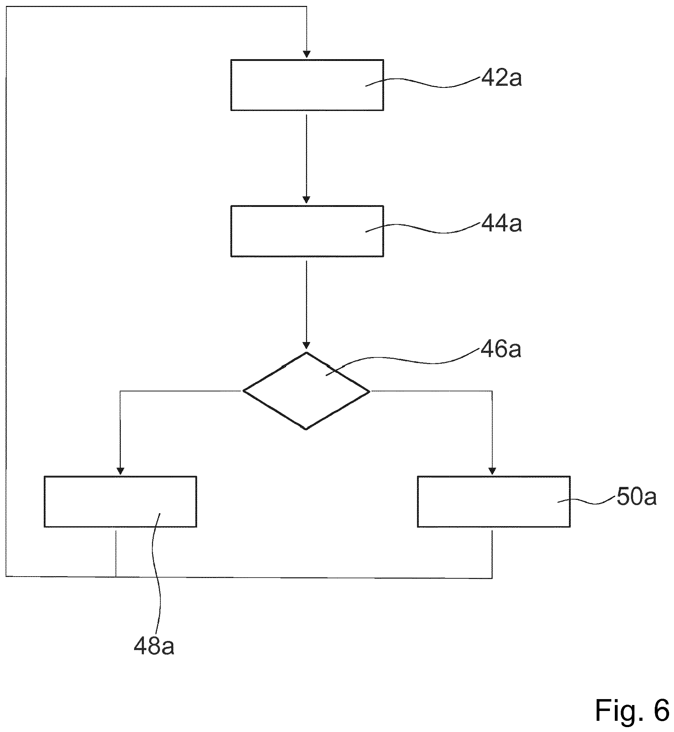

Figur 6 zeigt ein Ablaufdiagramm eines Verfahrens zu einem Betrieb der Schutzkassette 10a. Während des Verfahrens werden Betriebs- und Prozessparameter des externen Schweißgeräts 22a in der Schutzkassette 10a angezeigt. Dazu werden in einem ersten Verfahrensschritt 42a über die Kommunikationseinheit 30a der Schutzkassette 10a Betriebs- und Prozessparameter des externen Schweißgeräts 22a abgerufen, bzw. Parameter vom Schweißgerät 22a zur Schutzkassette 10a übertragen. Das Schweißgerät 22a ist hierbei der Initiant. Grundsätzlich wäre jedoch auch denkbar, dass die Daten aktiv von der Kommunikationseinheit 30a abgerufen werden. Grundsätzlich wäre auch denkbar, dass das derart abläuft, dass nur Daten von dem Schweißgerät 22a gesendet werden, wenn sich Parameter verändert haben. Anschließend werden in einem weiteren Verfahrensschritt 44a die abgerufenen Betriebs- und Prozessparameter des externen Schweißgeräts 22a von der Steuer- und Regeleinheit 18a der Schutzkassette 10a verarbeitet. Darauffolgend wird in einer Verzweigung 46a überprüft, ob ein Alarmzustand vorliegt. Liegt ein Alarmzustand vor, werden in einem weiteren Verfahrensschritt 48a die abstrahierten Symbolanzeigen 24a, 26a zu einer blinkenden Ausgabe eines Alarmmusters angesteuert. Liegt kein Alarmzustand vor, wird in einem weiteren Verfahrensschritt 50a die aktuell vorliegende Programmnummer des externen Schweißgeräts 22a codiert und über die erste abstrahierte Symbolanzeige 24a ausgegeben. Ferner wird in dem Verfahrensschritt 50a eine aktuell vorliegende Stromstärke und/oder ein anderer Parameter des externen Schweißgeräts 22a gemäß der Skalierungsvorschrift verarbeitet und über die zweite abstrahierte Symbolanzeige 26a ausgegeben. Nach dem Verfahrensschritt 50a oder dem Verfahrensschritt 48a wird der erste Verfahrensschritt 42a wiederholt.

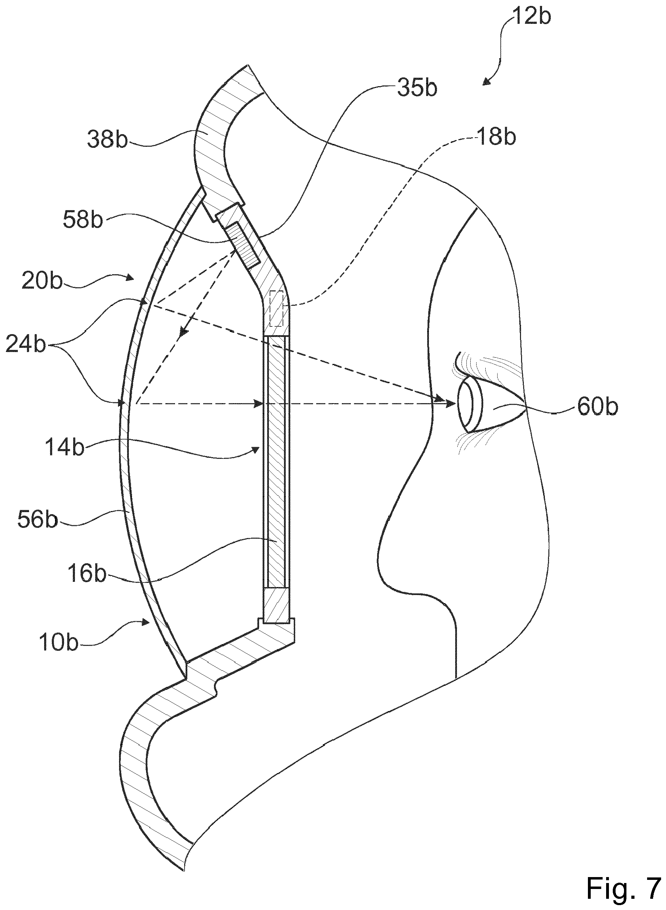

In der Figur 7 ist ein weiteres Ausführungsbeispiel der Erfindung gezeigt. Die nachfolgenden Beschreibungen beschränken sich im Wesentlichen auf die Unterschiede zwischen den Ausführungsbeispielen, wobei bezüglich gleichbleibender Bauteile, Merkmale und Funktionen auf die Beschreibung des Ausführungsbeispiels der Figuren 1 bis 6 verwiesen werden kann. Zur Unterscheidung der Ausführungsbeispiele ist der Buchstabe a in den Bezugszeichen des Ausführungsbeispiels in den Figuren 1 bis 6 durch den Buchstaben b in den Bezugszeichen des Ausführungsbeispiels der Figur 7 ersetzt. Bezüglich gleich bezeichneter Bauteile, insbesondere in Bezug auf Bauteile mit gleichen Bezugszeichen, kann grundsätzlich auch auf die Zeichnungen und/oder die Beschreibung des Ausführungsbeispiels der Figuren 1 bis 6 verwiesen werden.

Die Figur 7 zeigt eine Blendschutzvorrichtung 12b. Die Blendschutzvorrichtung 12b ist dazu vorgesehen, während eines Betriebs von einem Bediener auf dem Kopf getragen zu werden. Die Blendschutzvorrichtung 12a ist von einem Schweißhelm gebildet. Die Blendschutzvorrichtung 12b weist eine Schutzkassette 10b auf. Die Schutzkassette 10b weist einen optischen Blendschutzfilter 14b auf. Der optische Blendschutzfilter 14b weist eine Flüssigkristallzelle 16b auf. Ferner weist die Schutzkassette 10b eine Steuer- und Regeleinheit 18b auf. Die Steuer- und Regeleinheit 18b ist dazu vorgesehen, abhängig von einem erfassten Arbeitszustand und einer Lichteinstrahlung eine Durchlässigkeit des optischen Blendschutzfilters 14b zu steuern.

Ferner weist die Blendschutzvorrichtung 12b eine Schildeinheit 38b auf. Die Schutzkassette 10b ist fest in der Schildeinheit 38b aufgenommen. Die Blendschutzvorrichtung 12b weist ferner eine Vorsatzscheibe 56b auf. Die Schutzkassette 10b weist die Vorsatzscheibe 56b auf.

Ferner weist die Schutzkassette 10b eine Anzeigeeinheit 20b auf. Die Anzeigeeinheit 20b ist zu einer Anzeige von Betriebs- und Prozessparametern des externen Schweißgeräts 22b vorgesehen. Die Anzeigeeinheit 20b weist eine abstrahierte Symbolanzeige 24b auf. Die abstrahierte Symbolanzeige 24b ist zu einer Anzeige unterschiedlicher Betriebs- und Prozessparameter eines externen Schweißgeräts vorgesehen. Die abstrahierte Symbolanzeige 24b der Anzeigeeinheit 20b ist von einer Balkenanzeige gebildet. Die abstrahierte Symbolanzeige 24b ist von einer Reflektionsanzeige gebildet. Die abstrahierte Symbolanzeige 24b ist von einer Head-up-Display-Anzeige gebildet. Die abstrahierte Symbolanzeige 24b ist von einer Reflektionsanzeige gebildet, bei welcher die für einen Bediener sichtbare Anzeige von einem bildgebenden Teil der Anzeigeeinheit 20b beabstandet ausgebildet ist. Die Anzeigeeinheit 20b weist hierzu eine bildgebende Einheit 58b und eine Reflektionsfläche auf. Die bildgebende Einheit 58b ist auf einer dem Bediener abgewandten Seite der Schutzkassette 10b angeordnet. Die bildgebende Einheit 58b ist von einem Balkendisplay gebildet. Die bildgebende Einheit 58b weist n diskrete Balkenpunkte auf. Die Balkenpunkte sind jeweils voneinander getrennt. Die Balkenpunkte der bildgebenden Einheit 58b bilden jeweils einzelne Leuchtpunkte aus, welche beispielsweise mittels einer LED beleuchtbar sind. Ferner sind die Balkenpunkte jeweils in zwei verschiedenen Farben beleuchtbar. Die Balkenpunkte weisen daher drei mögliche Zustände auf: einen unbeleuchteten Zustand, einen Zustand, bei welchem der Balkenpunkt in einer ersten Farbe beleuchtet wird, und einen Zustand, bei welchem der Balkenpunkt in einer zweiten Farbe beleuchtet wird. Die abstrahierte Symbolanzeige 24b ist in einem Betrieb auf der Reflektionsfläche angeordnet. Die Reflektionsfläche ist von der Vorsatzscheibe 56b gebildet. Grundsätzlich wäre jedoch auch eine andere, einem Fachmann als sinnvoll erscheinende Ausbildung der Reflektionsfläche denkbar. Die Vorsatzscheibe 56b ist transparent ausgebildet, sodass der Bediener sowohl die gespiegelte abstrahierte Symbolanzeige 24b der Anzeige als auch die reale Welt hinter der Vorsatzscheibe 56b wahrnehmen kann. Die abstrahierte Symbolanzeige 24b kann dabei sowohl in einem zentralen Sichtfeld des Bedieners als auch in einem peripheren Sichtfeld des Bedieners angeordnet sein. Abhängig von einem Abstrahlwinkel der bildgebenden Einheit 58b kann die abstrahierte Symbolanzeige 24b entweder in dem zentralen Sichtfeld des Bedieners oder in einem peripheren Sichtfeld des Bedieners angeordnet werden. Dies kann vorzugsweise von dem Bediener manuell eingestellt werden. Die abstrahierte Symbolanzeige 24b ist in zumindest einem Betriebszustand in einem peripheren Sichtfeld des Bedieners angeordnet. Die abstrahierte Symbolanzeige 24b der Anzeigeeinheit 20b ist während eines Betriebs in zumindest einem Betriebszustand, zumindest bei gerader Kopfhaltung und einem direkten Geradeausschauen des Bedieners, in einem peripheren Sichtfeld angeordnet. Die Reflektion der bildgebenden Einheit 58b, welche von der abstrahierten Symbolanzeige 24b gebildet ist, fällt dabei dezentral auf die Augen 60b des Bedieners. Bei einer Anordnung der abstrahierten Symbolanzeige 24b im zentralen Sichtfeld des Bedieners fällt die Reflektion der bildgebenden Einheit 58b, welche von der abstrahierten Symbolanzeige 24b gebildet ist, zentral auf die Augen 60b des Bedieners. In der Figur 7 ist ein Weg des Lichts der bildgebenden Einheit 58b sowohl bei einer Anordnung der abstrahierten Symbolanzeige 24b in einem peripheren Sichtfeld als auch in einem zentralen Sichtfeld über eine gestrichelte Linie dargestellt.

## Patentansprüche

1. Schutzkassette für eine Blendschutzvorrichtung (12a; 12b), mit einem optischen Blendschutzfilter (14a; 14b), welcher zumindest eine Flüssigkristallzelle (16a; 16b) aufweist, mit zumindest einer Steuer- und/oder Regeleinheit (18a; 18b), welche dazu vorgesehen ist, abhängig von einem erfassten Arbeitszustand und/oder einer Lichteinstrahlung eine Durchlässigkeit des optischen Blendschutzfilters (14a; 14b) zu steuern und/oder zu regeln, und mit zumindest einer Anzeigeeinheit (20a; 20b) zu einer Anzeige von Betriebs- und/oder Prozessparametern eines externen Schweißgeräts (22a; 22b) und/oder eines Schweißverfahrens,
**dadurch gekennzeichnet, dass**
die zumindest eine Anzeigeeinheit (20a; 20b) zumindest eine abstrahierte Symbolanzeige (24a, 26a, 52a, 54a; 24b) aufweist, welche zumindest während eines Betriebs in einem peripheren Sichtfeld eines Bedieners angeordnet ist.

2. Schutzkassette zumindest nach dem Oberbegriff des Anspruchs 1, insbesondere nach Anspruch 1,
**dadurch gekennzeichnet, dass**
die zumindest eine Anzeigeeinheit (20b) zumindest eine abstrahierte Symbolanzeige (24b) aufweist, welche von einer Reflektionsanzeige gebildet ist.

3. Schutzkassette nach Anspruch 1 oder 2,
**dadurch gekennzeichnet, dass**
die zumindest eine Anzeigeeinheit (20a) zumindest zwei abstrahierte Symbolanzeigen (24a, 26a, 52a, 54a) aufweist, welche zumindest während eines Betriebs jeweils in einem peripheren Sichtfeld des Bedieners angeordnet sind.

4. Schutzkassette nach einem der vorhergehenden Ansprüche,
**dadurch gekennzeichnet, dass**
die zumindest eine abstrahierte Symbolanzeige (24a, 26a, 52a, 54a) der Anzeigeeinheit (20a) während eines Betriebs in einem unfokussierbaren Sichtfeld des Bedieners angeordnet ist.

5. Schutzkassette nach einem der vorhergehenden Ansprüche,
**dadurch gekennzeichnet, dass**
die zumindest eine abstrahierte Symbolanzeige (24a, 26a, 52a, 54a; 24b) der Anzeigeeinheit (20a) von einer Balken- oder Punktanzeige gebildet ist.

6. Schutzkassette nach Anspruch 5,
**dadurch gekennzeichnet, dass**
die zumindest eine als Balken- oder Punktanzeige ausgebildete abstrahierte Symbolanzeige (26a) der Anzeigeeinheit (20a), welche n diskrete Balkenpunkte (28a) oder Punkte besitzt, gemäß einer Skalierungsvorschrift zu einer Anzeige eines Werts eines Betriebs- und/oder Prozessparameters des externen Schweißgeräts (22a) als Balkenpunkt (28a) oder Punkt zwischen den Maximalwerten vorgesehen ist.

7. Schutzkassette zumindest nach Anspruch 5,
**dadurch gekennzeichnet, dass**
die zumindest eine als Balken- oder Punktanzeige ausgebildete abstrahierte Symbolanzeige (26a) der Anzeigeeinheit (20a) zumindest einen andersfarbigen Balkenpunkt (28a) oder Punkt aufweist, welcher als Orientierungshilfe dient.

8. Schutzkassette nach einem der vorhergehenden Ansprüche,
**dadurch gekennzeichnet, dass**
die zumindest eine abstrahierte Symbolanzeige (24a; 24b) der Anzeigeeinheit (20a; 20b) dazu vorgesehen ist, einen zu einer Anzeige vorgesehenen Wert eines Betriebs und/oder Prozessparameters des externen Schweißgeräts (22a) codiert darzustellen.

9. Schutzkassette zumindest nach Anspruch 5,
**dadurch gekennzeichnet, dass**
die zumindest eine als Balken- oder Punktanzeige ausgebildete abstrahierte Symbolanzeige (24a; 24b) der Anzeigeeinheit (20a; 20b) dazu vorgesehen ist, einen zu einer Anzeige vorgesehenen Wert durch eine Folge von langen und/oder kurzen Balkensegmenten oder Punktfolgen darzustellen.

10. Schutzkassette nach einem der vorhergehenden Ansprüche,
**dadurch gekennzeichnet, dass**
die zumindest eine abstrahierte Symbolanzeige (24a, 26a; 24b) der Anzeigeeinheit (20a; 20b) dazu vorgesehen ist, einen Alarmzustand auszugeben.

11. Schutzkassette nach Anspruch 10,
**dadurch gekennzeichnet, dass**
die zumindest eine abstrahierte Symbolanzeige (24a, 26a; 24b) der Anzeigeeinheit (20a; 20b) dazu vorgesehen ist, einen Alarmzustand durch die Ausgabe eines blinkenden Alarmmusters auszugeben.

12. Schutzkassette nach einem der vorhergehenden Ansprüche,
**gekennzeichnet durch**
zumindest eine Kommunikationseinheit (30a) zu einer Datenkommunikation mit dem externen Schweißgerät (22a).

13. Blendschutzvorrichtung mit einer Schutzkassette (10a; 10b) nach einem der vorhergehenden Ansprüche.

14. System mit einem externen Schweißgerät (22a) und mit einer Blendschutzvorrichtung (12a; 12b) nach Anspruch 13.

15. Verfahren zum Betrieb einer Schutzkassette (10a; 10b) nach einem der Ansprüche 1 bis 12.
